# EUROPEAN PATENT SPECIFICATION

(11) **EP 2 459 185 B1**
(45) Date of publication and mention of the grant of the patent: **27.11.2013**
(21) Application number: 10742421.0
(22) Date of filing: 22.07.2010
(51) Int. Cl.: A61K 33/30, A61K 31/197, A61K 31/385, A61K 31/417, A61K 31/4172, A61K 31/4406, A61K 31/4415, A61K 31/51, A61K 31/519, A61K 31/525, A61K 31/714, A61P 25/02

(54) **COMPOSITION COMPRISING ALPHA-LIPOIC ACID AND CARNOSINE FOR TREATING THE PHANTOM LIMB SYNDROME**
ZUSAMMENSETZUNG MIT EINER ALPHA-LIPONSÄURE UND CARNOSIN ZUR BEHANDLUNG VON PHANTOMSCHMERZ
COMPOSITION COMPRENANT DE L'ACIDE ALPHA-LIPOÏQUE ET DE LA CARNOSINE POUR TRAITER LE SYNDROME DU MEMBRE FANTÔME

(30) Priority: 30.07.2009 IT MI20091377
(43) Date of publication of application: 06.06.2012
(73) Proprietor: Difass International S.r.l., 59100 Prato (IT)
(72) Inventor: GUASTI, Pier, Luigi, I-47838 Riccione (RN) (IT)
(74) Representative: Minoja, Fabrizio
(86) International application number: PCT/EP2010/004479
(87) International publication number: WO 2011/012256

(56) References cited:
- WO-A1-2008/094664
- WO-A2-2008/015535
- US-A- 6 117 900
- STREMMEL ET AL: "The Impact of Immunological Parameters on the Development of Phantom Pain after Major Amputation" EUROPEAN JOURNAL OF VASCULAR AND ENDOVASCULAR SURGERY, SAUNDERS, LONDON, GB, vol. 30, no. 1, 1 July 2005 (2005-07-01), pages 79-82, XP005043212 ISSN: 1078-5884
- KAMEI J ET AL: "Preventive effect of l-carnosine on changes in the thermal nociceptive threshold in streptozotocin-induced diabetic mice" EUROPEAN JOURNAL OF PHARMACOLOGY, ELSEVIER BV, NL LNKD- DOI:10.1016/J.EJPHAR.2008.10.002, vol. 600, no. 1-3, 14 December 2008 (2008-12-14), pages 83-86, XP025672088 ISSN: 0014-2999 [retrieved on 2008-10-09]
- JUAN J ARCHELOS: "Treatment options in painful diabetic polyneuropathy" WIENER KLINISCHE WOCHENSCHRIFT ; THE MIDDLE EUROPEAN JOURNAL OF MEDICINE, SPRINGER-VERLAG, VI LNKD- DOI:10.1007/S00508-007-0795-2, vol. 119, no. 7-8, 1 May 2007 (2007-05-01) , pages 205-216, XP019518571 ISSN: 1613-7671

## Description

### Background of the invention.

In a general aspect thereof, the present invention relates to a composition comprising α-lipoic acid and carnosine, or pharmaceutically acceptable derivatives thereof selected from α-lipoic acid salts with alkali metals, α-lipoic acid salts with alkaline earth metals, α-lipoic acid salts with transition metals, a salt of α-lipoic acid with 2-amino-2-hydroxymethylpropane-1,3-diol in a 1:1 ratio, methyl-lipoate, ethyl-lipoate, or lipoamides, and a salt of carnosine, anserine, homocarnosine, N-α-acetylcarnosine, or carcinine for treating the phantom limb syndrome.

Within the framework of the present description and of the appended claims, the term "phantom limb syndrome" is used to indicate a syndrome related to the abnormal persisting sensation of the limb after its amputation.

More specifically, for the purposes of the present invention, the term "phantom limb syndrome" is used to indicate a syndrome including the following components according to what was suggested by Jensen and Rasmussen (Textbook of Pain, 3rd edition. Churchill Livingstone, New York. 1997; 861-5):
1) a "phantom limb pain" component, which is any painful sensations that are referred to the absent limb;
2) a "phantom limb sensation" component, which is any sensations that are referred to the absent limb, except pain; and
3) a "stump pain" component, which is pain localized to the stump ("residual limb pain").

The great majority of upper limb amputations is due to accidents. A small percentage is carried out to treat a disease (e.g. malignant tumor removal). The amputations of a lower limb occur, almost in the same percentage, as after-effect of an accident or surgical operation carried out in order to treat the consequences of a medical problem, for example as a consequence of insufficient circulation due to diabetes.

After the amputation of an upper or lower limb, it may appear the sensation defined "phantom limb", in which the patient feels that he still has the amputated limb (e.g. at night, he gets up and falls on the ground).

These sensations defined as "phantom limb sensation" can be of painful or non-painful type. The phantom limb pain falls within the category of neuropathic pain or also "deafferentation pain", due to the partial or complete interruption of the peripheral or central afferent neural activity.

The syndrome in general, and the painful type in particular, cannot be caused by a limb problem, but by a modification of the nervous system upstream of the seat of amputation: the brain codes in an altered way the nervous signals as if they came from the amputated limb. The pain can simulate a sensation of squeezing, burning or compressing, but often it is different from any other previous sensation.

The incidence of phantom limb pain has an extremely wide range, from 2 to 98%, even if more recent studies suggest that 60-80% of the amputees feel phantom pain (Jensen TS, Nikolajsen L., Textbook of Pain, 4th edition Churchill Livingstone, New York. 1999; 799-814), while 80-100% of these patients feel the phantom limb sensation. Even the duration is variable, ranging from a few months to many years, even if in most cases it decreases in a two-year period. When the phantom limb becomes the center of intense pain, it is a serious obstacle to the completion of prosthesis rehabilitation.

The mechanisms underlying the phantom limb sensation are not currently understood, even if various theories have been proposed. The difficulty of treatment is linked to the lack of understanding of the pathogenic mechanisms. There is clear evidence that cortical reorganization is associated with phantom limb pain, although perhaps not phantom limb sensation (Woodhouse A., Clinical and Experimental Pharmacology and Physiology (2005) 32, 132-134).

The treatments proposed up to now for this kind of problem have not led to definite results. The medical treatment of amputee pain, also comprising the phantom limb pain, presents controversial results.

### Related art

In literature, the effectiveness is known of the use of a neuromodulator having gabapentin (1-(aminomethyl)cyclohexane acetic acid) as active principle in the phantom limb pain treatment (D.G. Smith et al. "Efficacy of gabapentin in treating chronic phantom limb and residual limb pain", Journal of Rehabilitation Research & Development, Vol. 42, No. 5, p. 645-654, 2005).

It is also known that an effective reduction of the phantom limb pain is achieved by using methadone (see, for example, L. Bergmans et al., "Methadone for phantom limb pain", The Clinical Journal of Pain, Vol. 18(3), pp. 203-205, 2002), or by using tricyclic anti-depressants (Kazuhiro Sato, "Management of phantom limb pain and sensation with Milnacipran", J. Neuropsychiatry Clin. Neurosci. Vol. 20:3, p. 368, 2008).

To treat pain syndrome, massages, laser therapy, electrical stimulation/TENS, etc. or treatments based on the use of α-lipoic acid (literature documents on this subject are not however available), are also commonly practiced.

US 6117900 discloses retigabine with an anticonvulsive and analgesic action, for the treatment of phantom limb pain. Additionally, this document discloses that neurodegenerative nerve damage and the underlying microangiopathy can be treated by the use of nerve cell protecting (neuroprotective) substances such as alpha-lipoic acid or other antioxidants such as vitamins relevant to nervous system, such as B1, B6 or B12. US 6117900 mentions that by improving a nerve function, it is possible for the pain sensation to subside in the long term.

Nevertheless, up to now, the mechanisms underlying phantom limb pain are still entirely hypothetical and a standardized treatment protocol, accredited on an international level, does not exist. Medical-pharmacological treatment is more concentrated on resolving or decreasing pain symptoms rather than the non-painful sensation; this in order to allow an improvement of the general kinesthesia for the purpose of a successful rehabilitation.

On the other hand, the perception of the phantom limb is strongly related to body image disorders: a negative perception of one's body image (evaluated by the administration of tests such as Abis and McGill Pain Questionnaire) leads to poor psychosocial adaptation and difficulties in the interpersonal sphere. Therefore, patients that have phantom limb experience are more subjected to emotional disorders, are more depressed and easily susceptible to stress. This hampers, delays and strongly affects the rehabilitation and psychological recovery of the patient. Quality of life should be considered the main objective of rehabilitation, which must therefore also include among its instruments treating remedies for solving all those disorders which compromise the psychological adaptation of the amputee patient, in particular the frequent perception of the phantom limb. It is thus necessary to evaluate and treat all the symptoms of the phantom limb syndrome.

### Summary of the invention

The Applicant has found that the solutions proposed for reducing phantom limb sensation in patients affected by this disorder and for reducing the outbreak of both phantom limb pain and stump pain, consequently improving the quality of life of patients affected by such symptoms, are not yet fully satisfying.

The problem underlying the present invention is therefore that of devising and providing new substances and compositions which are useful to treat the phantom limb syndrome and, more particularly, to achieve a reduction of phantom limb sensation, achieving at the same time also a desired reduction of pain outbreak.

In this regard, the Applicant has surprisingly found that it is possible to achieve these desired effects thanks to the administration of a composition comprising α-lipoic acid and carnosine to patients affected by phantom limb sensation.

More particularly, according to a first aspect, the present invention relates to a composition comprising α-lipoic acid and carnosine, or pharmaceutically acceptable derivatives thereof as defined in the present claim 1, for use in the treatment of phantom limb syndrome.

According to the invention, in fact, it was found that a composition comprising α-lipoic acid and carnosine, or pharmaceutically acceptable derivatives thereof as defined in the present claim 1, is surprisingly capable to achieve an unexpected improvement in the treatment of the phantom limb syndrome in particular both with regard to phantom limb sensation and for reducing the pain component of the phantom limb syndrome, with a stabilization of the results in time. In addition, the proposed treatment is very well tolerated, has no side effects and has complementary benefits such as antioxidant and anti-glycan effects.

Within the framework of the present description and of the appended claims, the term "treatment", is used to indicate the reduction of the severity and frequency of the symptoms, the disappearance of the symptoms, the prevention of the symptoms and of the causes originating them with a general improvement in the quality of life.

Within the framework of the present description and of the appended claims, the term α-lipoic acid, is used to indicate both α-lipoic acid as such (IUPAC name: 5-(1,2-thiolan-3-yl)pentanoic acid; CAS number 62-46-4, 1077-28-7 or 1200-22-2), and its reduced form, also known with the name of dihydrolipoic acid (IUPAC name, 6,8-bis-sulfanyloctanoic acid; CAS number 462-20-4), wherein the sulfur atoms are present as free thiols (-SH), and both the enantiomeric forms of α-lipoic acid and dihydrolipoic acid (form R and form L), as well as the racemic form and their mixtures in any proportion. Pharmaceutically acceptable derivatives of α-lipoic acid are selected from salts, esters, amides, as well as all mixtures thereof. These substances may be selected from the group comprising: α-lipoic salts with alkali metals (such as sodium lipoate and potassium lipoate), with alkaline earth metals (such as magnesium lipoate), with transition metals (such as zinc lipoate), salts with amines (such as the salt of α-lipoic acid with 2-amino-2-hydroxymethylpropane-1,3-diol in a 1:1 ratio), esters with aliphatic carboxylic acids (such as methyl-lipoate and ethyl-lipoate), amides with amines (such as lipoamides).

Within the framework of the present description and of the appended claims, the term carnosine is used to indicate the compound N-β-alanyl-histidine (IUPAC name: 2-(3-aminopropanoylamino)-3-(3H-imidazol-4-yl)propanoic acid; CAS number 305-84-0 or 7683-28-5), both the enantiomeric forms of D-Carnosine (N-β-alanyl-D-histidine) and L-Carnosine (N-β-alanyl-L-histidine), as well as the racemic form and mixtures thereof in any proportion. Pharmaceutically acceptable derivatives of carnosine are selected from a salt of carnosine, e.g. with zinc, copper or manganese, or derivatives, such as anserine (methylated form of carnosine), homocarnosine, N-α-acetylcarnosine, carcinine.

Carnosine is preferably used in the non-complexed form.

In a preferred embodiment, the composition to be used for the purposes of the invention comprises, for each daily dosage amount, α-lipoic acid or a pharmaceutically acceptable derivative thereof as defined in the present claim 1 in an amount of 150 to 1,400 mg and carnosine or a pharmaceutically acceptable derivative thereof as defined in the present claim 1 in an amount of 50 to 600 mg, more preferably α-lipoic acid or a pharmaceutically acceptable derivative thereof as defined in the present claim 1 in an amount of 250 to 1,200 mg, carnosine or a pharmaceutically acceptable derivative thereof as defined in the present claim 1 in an amount of 100 to 500 mg, and still more preferably, α-lipoic acid or a pharmaceutically acceptable derivative thereof as defined in the present claim 1 in an amount of 350 to 900 mg and carnosine or a pharmaceutically acceptable derivative thereof as defined in the present claim 1 in an amount of 150 to 450 mg.

Within the framework of the present description and of the appended claims, the term "daily dosage amount" of an active ingredient is used to indicate the total amount of the single active ingredient taken in one day (once or several times a day), by means of the various dosage forms. The daily dosage amounts and the relative proportions are based on the content of active ingredient such that an appropriate conversion is necessary when the ingredient is in salt or derivative form.

For the purposes of the present description and the appended claims, except where otherwise indicated, all numerical entities expressing amounts, parameters, percentages, and so forth, are to be understood as being preceded in all instances by the term "about". Also, all ranges of numerical entities include all the possible combinations of the maximum and minimum numerical values and all the possible intermediate ranges therein, in addition to those specifically indicated below.

Within the framework of the present invention, it was also found that it is possible to achieve a particularly effective result in the treatment of the phantom limb syndrome when the various ingredients of the composition are present in specific weight ratios with respect to each other.

Preferably, the composition to be used for the purposes of the invention comprises, for each daily dosage amount, α-lipoic acid and carnosine or pharmaceutically acceptable derivatives thereof as defined in the present claim 1 in a ratio from 0.4:1 to 5:1 in parts by weight, preferably in a ratio from 1:1 to 2:1 in parts by weight.

In a preferred embodiment, the composition to be used for the purposes of the invention further comprises, for each daily dosage amount, an effective amount of zinc-based compounds.

In this way, a further increase of the reduction of the phantom limb sensation, as well as a further reduction of the painful component of the phantom limb syndrome, is achieved.

By way of example, the zinc-based compounds may be selected from those commonly used in the art, such as for example , zinc acetate, zinc chloride, zinc citrate, zinc gluconate, zinc pidolate, zinc lactate, zinc oxide, zinc carbonate, zinc sulfate, zinc aspartate, zinc ascorbate, zinc phosphate, zinc stearate, zinc methionine, and the like. Among these, zinc gluconate and zinc pidolate have in particular a preferred and advantageous use.

Preferably, the composition to be used for the purposes of the invention comprises, for each daily dosage, elementary zinc in an amount from 1 to 100 mg.

Within the framework of this preferred embodiment, the composition to be used for the purposes of the invention comprises, for each daily dosage, α-lipoic acid or a pharmaceutically acceptable derivative thereof as defined in the present claim 1 in an amount of 150 to 1,400 mg, carnosine or a pharmaceutically acceptable derivative thereof as defined in the present claim 1 in an amount of 50 to 600 mg, and a zinc-based compound wherein the amount of elementary zinc is from 1 to 100 mg; more preferably, α-lipoic acid or a pharmaceutically acceptable derivative thereof as defined in the present claim 1 in an amount of 250 to 1,200 mg, carnosine or a pharmaceutically acceptable derivative thereof as defined in the present claim 1 in an amount of 100 to 500 mg, and a zinc-based compound in which the amount of elementary zinc is from 3 to 75 mg; still more preferably, α-lipoic acid or a pharmaceutically acceptable derivative thereof as defined in the present claim 1 in an amount of 350 to 900 mg, carnosine or a pharmaceutically acceptable derivative thereof as defined in the present claim 1 in an amount of 150 to 450 mg, and a zinc-based compound in which the amount of elementary zinc is from 5 to 50 mg.

In such a preferred embodiment, the composition to be used for the purposes of the invention comprises, for each daily dosage amount, α-lipoic acid or a pharmaceutically acceptable derivative thereof as defined in the present claim 1, carnosine or a pharmaceutically acceptable derivative thereof as defined in the present claim 1 and a zinc-based compound wherein the ratio between α-lipoic acid or a pharmaceutically acceptable derivative thereof as defined in the present claim 1 and elementary zinc is from 5.1 to 400:1 in parts by weight, more preferably from 10:1 to 200:1.

In such a preferred embodiment, the composition to be used for the purposes of the invention comprises, for each daily dosage amount, α-lipoic acid or a pharmaceutically acceptable derivative thereof as defined in the present claim 1, carnosine or a pharmaceutically acceptable derivative thereof as defined in the present claim 1 and a zinc-based compound wherein the ratio between carnosine or a pharmaceutically acceptable derivative thereof and elementary zinc is from 5:1 to 50:1 in parts by weight; more preferably from 15:1 to 30:1.

Preferably, the composition to be used for the purposes of the invention also incorporates, for each dosage unit, at least one vitamin, such as for example thiamine (vitamin B1), riboflavin (vitamin B2), pyridoxine (vitamin B6), pantothenic acid (vitamin B5), cyanocobalamin (vitamin B12), niacin (or nicotinamide, otherwise known as vitamin B3 or PP) and folates.

In this way, the antioxidant, nutritional and physiological properties are improved.

The composition to be used for the purposes of the invention can be formulated in solid or liquid form as a function of the nature of the various ingredients used by employing preparation techniques and methods known to those skilled in the art.

The composition to be used for the purposes of the invention can therefore be advantageously formulated in the form of a powder, micro-granule, granule, microcapsule, both with immediate release and with a modified release and in mixed form (part of the formulation with immediate release and part with modified or delayed release), where the technology for the modified release relates to the release of the active principle from the matrix with slower kinetics than the immediate release formulations, due to the chemical-physical properties of the excipients which constitute the same, so that it may be possible to influence the absorption kinetics of the molecule of interest in a controlled and rational manner.

The advantages of the modified release formulations mainly lie in the reduction of the number of daily administrations to the patient and in the possibility of administering products with low half-life for prolonged times, extending their effectiveness time span with respect to a conventional formulation.

Preferably, the composition to be used for the purposes of the invention is in the form of a powder, micro-granule, granule, micro-capsule, rigid capsule, soft capsule, tablet or pill, bag, aqueous solution, oily solution, suspension or oral drops.

Such formulations may be prepared by suitably selecting the various ingredients in the appropriate physical form, according to the knowledge of those skilled in the art. Thus, for example, the preferred composition in the form of a powder, micro-granule, granule and micro-capsule may be packaged for oral use in the form of tablet or pill, rigid capsule, soft capsule, bag, aqueous solution, oily solution, suspension or oral drops.

Preferably, the composition to be used for the purposes of the invention is in the form of tablet, hard gelatin capsule, soft gelatin capsule wherein the active principles are dispersed in an oily carrier, or in bags.

Preferably, the composition to be used for the purposes of the invention also incorporates, for each dosage unit, at least one pharmaceutically acceptable excipient, preferably an excipient selected from those commonly used in the art of formulating the compositions used in the invention.

Advantageously, such excipients, depending upon the administration form of the composition to be used for the purposes of the invention, allow to achieve a good workability and free flowing properties of the powders, a good hardness of the tablet, a good dispersion of the ingredients in the liquids, a suitable time for disintegration and dissolution, or good organoleptic characteristics of the finished product, such as taste, odor and color.

In particular, among them the following find a preferred and advantageous use for manufacturing tablets of the composition to be used for the purposes of the present invention: microcrystalline cellulose, such as hydroxypropyl methylcellulose, or dibasic calcium phosphate, as fillers, magnesium stearate as lubricant for the flowability of the powder, and colloidal silica or talc for improving the workability of the powder at the time of compression.

According to a second aspect, the present invention relates to a composition as described above for use in treating the phantom limb syndrome.

According to a third aspect, the present invention relates to the use of a composition as described above for manufacturing a medicament for treating the phantom limb syndrome.

Additional characteristics and advantages of the invention will be better apparent from the following description of some examples of compositions according to the invention.

### EXAMPLES

### Example 1.

Compositions 1-2 (invention). With methods known *per se,* two compositions in tablet form having the active ingredients indicated in Table 1 were prepared from commercially available ingredients in powder form.

Each single component was weighed on a suitable scale; when necessary the powder was sieved. The weighed powder was introduced into the mixer where it remained for the time necessary for obtaining perfect homogenization. Then, it was unloaded in a suitable container for food use. The powder was introduced into the hopper of a tabletting apparatus in order to be compressed and transformed in tablet form. Once all the parameters of the tabletting apparatus were adjusted, so as to have a tablet with the required hardness and weight, the production process for obtaining the tablets was started. The tablets were then stored in suitable containers for food use until packaging.

**Table I**

| Ingredients | Composition 1 | Composition 2 |
|---|---|---|
| | (mg) | (mg) |
| | (invention) | (invention) |
| α-lipoic acid | 600 | 600 |
| Carnosine | 165 | 165 |
| Zinc citrate | | 23.97 |
| Pantothenic acid (Vit. B5) | 3 | 3 |
| Pyridoxine (Vit. B6) | 1 | 1 |
| Riboflavin (Vit. B2) | 0.8 | 0.8 |
| Thiamine (Vit. B1) | 0.7 | 0.7 |
| Cyanocobalamin (Vit. B12) | 0.005 | 0.005 |
| Nicotinamide (vitamin B3 or PP) | 9 | 9 |
| Folates | 0.1 | 0.1 |

### Example 2

Three groups, A, B and C, were formed, each with 5 patients of both sexes who underwent the amputation of the lower limb and were affected by phantom limb syndrome; the groups were uniform and comparable with each other. In order not to negatively influence the test, it was decided not to include the following in such groups: diabetic patients (in order to avoid interferences with the symptoms of diabetic neuropathy and with the drugs used by the diabetic patient), subjects with psychiatric pathologies also at a compensation stage, subjects with serious cognitive deficits, subjects affected by active hepatic and/or renal pathologies, subjects with organ pathologies and/or other serious comorbidities.

During the period in which the patients were subjected to the tests, they did not vary their lifestyle (diet, physical activity and/or athletic activity, medical treatments underway).

Group A (reference) was subdivided into two uniform groups A1 and A2; group A1 was subjected to a conventional treatment, orally taking for two months a daily dosage amount of the neuromodulator Gabapentin, a known anti-epileptic drug with indications in the treatment of neuropathic pain, as described in US patent application publication No. US 2006/159743. Group A2 was subjected to the same treatment, substituting the neuromodulator Gabapentin with the analogous neuromodulator Pregabalin.

Groups B and C were subjected, during the same two-month period, to a daily uptake of the composition 1 and, respectively, 2 of the present invention, in the same daily dosage amount of 1,200 mg.

At the time the test was started, (t0), after 30 days (t1) and after 60 days (t2) from the start of the tests, the following parameters were measured for each patient:
- the qualitative-quantitative evaluation of the sensation of possession of the missing limb (phantom limb sensation), by means of ABIS (Amputee Body Image Scale), as described for example by P. Gallagher et al. in "American Journal of Physical Medicine & Rehabilitation" 86, 3 205-215, 2007. This text is aimed at investigating how an amputee perceives his own body image. It is constituted by 20 points; the subjects are asked to indicate their answer by using a scale from 1 (never) to 5 (always). The scale produces scores from 20 to 100, and high scores indicate a serious disorder of the body image;

- the subjective perception of the phantom limb pain and stump pain (residual limb pain), by means of a visual analogue scale (VAS), as described, for example, by D. Gould et al. in "Journal of Clinical Nursing" 10, 697-706, 2001;
- the significant pain felt by patients over time by means of the method called "McGill Pain Questionnaire", as described for example by Melzack R. in "The McGill Pain Questionnaire: Major properties and scoring methods". Pain, 1975, 1: 277-299;
- the quality of life (QoL), by means of a questionnaire including 36 questions which allowed to understand the impact of a disease on various aspects of Quality of Life: physical activity, limitations due to physical health, emotional state, physical pain, perception of the general health state, vitality, social activities, mental health, change of health state.

From the results of such tests, remarkable improvements in the treatment of the phantom limb syndrome could only be observed for the patients of groups B and C. The patients of groups A, treated with techniques known in the art (gabapentin/pregabalin), in fact, only experienced a certain decrease of the pain, but did not receive any benefit with regard to the phantom limb sensation. On the other hand, the patients of group B subjected to a daily oral administration of composition 1 of the present invention comprising α-lipoic acid and carnosine and, in an even clearer manner, the patients of group C, subjected to a daily oral administration of composition 2 of the present invention comprising α-lipoic acid, carnosine and zinc-based compounds, showed unexpected positive effects on the phantom limb syndrome, not only with regard to the reduction of phantom limb pain and stump pain (by means of the VAS and McJill Questionnaire evaluation scales) and the stabilization of the results in time, but surprisingly also for the disappearance of the phantom limb sensation, contributing to substantially improve the quality of life of the patients subjected to such a treatment.

## Claims

1. Composition comprising α-lipoic acid or a derivative thereof selected from α-lipoic acid salts with alkali metals, α-lipoic acid salts with alkaline earth metals, α-lipoic acid salts with transition metals, a salt of α-lipoic acid with 2-amino-2-hydroxymethylpropane-1,3-diol in a 1:1 ratio, methyl-lipoate, ethyl-lipoate, or lipoamides and carnosine or pharmaceutically acceptable derivatives thereof selected from a salt of carnosine, anserine, homocarnosine, N-α-acetylcarnosine, or carcinine for use in treating the phantom limb syndrome.

2. Composition for use according to claim 1 comprising, for each daily dosage amount, α-lipoic acid or a pharmaceutically acceptable derivative thereof in an amount of 150 to 1,400 mg and carnosine or a pharmaceutically acceptable derivative thereof in an amount of 50 to 600 mg.

3. Composition for use according to claim 2 comprising, for each daily dosage amount, α-lipoic acid or a pharmaceutically acceptable derivative thereof in an amount of 350 to 900 mg and carnosine or a pharmaceutically acceptable derivative thereof in an amount of 150 to 450 mg.

4. Composition for use according to claim 1, 2 or 3 comprising, for each daily dosage amount, α-lipoic acid and carnosine or pharmaceutically acceptable derivatives thereof in a ratio from 0.4:1 to 5:1 in parts by weight.

5. Composition for use according to any one of claims 1 to 4 further comprising an effective amount of zinc-based compounds.

6. Composition for use according to claim 5 comprising, for each daily dosage amount, elementary zinc in an amount from 1 to 100 mg.

7. Composition for use according to claim 5 comprising, for each daily dosage amount, α-lipoic acid or a pharmaceutically acceptable derivative thereof in an amount of 150 to 1,400 mg, carnosine or a pharmaceutically acceptable derivative thereof in an amount of 50 to 600 mg, and a zinc-based compound wherein the amount of elementary zinc is from 1 to 100 mg.

8. Composition for use according to any one of claims 5 to 7 comprising, for each daily dosage amount, α-lipoic acid or a pharmaceutically acceptable derivative thereof, carnosine or a pharmaceutically acceptable derivative thereof and a zinc-based compound wherein the ratio between carnosine or a pharmaceutically acceptable derivative thereof and elementary zinc is from 5:1 to 50:1 in parts by weight.

9. Composition for use according to any one of claims 5 to 8 comprising, for each daily dosage amount, α-lipoic acid or a pharmaceutically acceptable derivative thereof, carnosine or a pharmaceutically acceptable derivative thereof and a zinc-based compound wherein the ratio between α-lipoic acid or a pharmaceutically acceptable derivative thereof and elementary zinc is from 5:1 to 400:1 in parts by weight.

10. Composition for use according to any one of the preceding claims comprising, for each daily dosage unit, at least one pharmaceutically acceptable excipient.

11. Composition for use according to any one of the preceding claims comprising, for each daily dosage unit, at least a vitamin, such as for example, thiamine (vitamin B1), riboflavin (vitamin B2), pyridoxine (vitamin B6), pantothenic acid (vitamin B5), cyanocobalamin (vitamin B12), niacin (vitamin B3) and folates.

12. Composition for use according to any one of the preceding claims in form of powder, micro-granule, granule, micro-capsule, rigid capsule, soft capsule, tablet or pill, bag, aqueous solution, oily solution, suspension or oral drops.

## Patentansprüche

1. Zusammensetzung umfassend α-Liponsäure oder eines ihrer Derivate ausgewählt aus Salzen der α-Liponsäure mit Alkalimetallen, Salzen der α-Liponsäure mit Erdalkalimetallen, Salzen der α-Liponsäure mit Übergangsmetallen, einem Salz der α-Liponsäure mit 2-Amino-2-hydroxymethylpropan-1,3-diol in einem Verhältnis 1:1, Methyllipoat, Ethyllipoat, oder Lipoamide und Carnosin oder seine pharmazeutisch annehmbaren Derivate ausgewählt aus einem Salz von Carnosin, Anserin, Homocarnosin, N-α-Acetylcarnosin oder Carcinin zur Verwendung in der Behandlung des Phantomgliedsyndroms.

2. Zusammensetzung zur Verwendung gemäß Anspruch 1, die für jede tägliche Dosierungsmenge α-Liponsäure oder eines ihrer pharmazeutisch annehmbaren Derivate in einer Menge von 150 bis 1.400 mg und Carnosin oder eines seiner pharmazeutisch annehmbaren Derivate in einer Menge von 50 bis 600 mg umfasst.

3. Zusammensetzung zur Verwendung gemäß Anspruch 2, die für jede tägliche Dosierungsmenge α-Liponsäure oder eines ihrer pharmazeutisch annehmbaren Derivate in einer Menge von 350 bis 900 mg und Carnosin oder eines seiner pharmazeutisch annehmbaren Derivate in einer Menge von 150 bis 450 mg umfasst.

4. Zusammensetzung zur Verwendung gemäß Anspruch 1, 2 oder 3, die für jede tägliche Dosierungsmenge, α-Liponsäure und Carnosin oder ihre pharmazeutisch annehmbaren Derivate in einem Verhältnis von 0,4:1 bis 5:1 in Gewichtsteilen umfasst.

5. Zusammensetzung zur Verwendung gemäß wenigstens einem der Ansprüche 1 bis 4, die zusätzlich eine wirksame Menge einer Zink-basierten Verbindung umfasst.

6. Zusammensetzung zur Verwendung gemäß Anspruch 5, die für jede tägliche Dosierungsmenge elementares Zink in einer Menge von 1 bis 100 mg umfasst.

7. Zusammensetzung zur Verwendung gemäß Anspruch 5, die für jede tägliche Dosierungsmenge α-Liponsäure oder eines ihrer pharmazeutisch annehmbaren Derivate in einer Menge von 150 bis 1.400 mg, Carnosin oder eines seiner pharmazeutisch annehmbaren Derivate in einer Menge von 50 bis 600 mg und eine Zink-basierte Verbindung, wobei die Menge an elementarem Zink 1 bis 100 mg beträgt, umfasst.

8. Zusammensetzung zur Verwendung gemäß wenigstens einem der Ansprüche 5 bis 7, die für jede tägliche Dosierungsmenge α-Liponsäure oder eines ihrer pharmazeutisch annehmbaren Derivate, Carnosin oder eines seiner pharmazeutisch annehmbaren Derivate und eine Zink-basierte Verbindung umfasst, wobei das Verhältnis zwischen Carnosin oder eines seiner pharmazeutisch annehmbaren Derivate und elementarem Zink von 5:1 bis 50:1 in Gewichtsteilen beträgt.

9. Zusammensetzung zur Verwendung gemäß wenigstens einem der Ansprüche 5 bis 8, die für jede tägliche Darreichungsform α-Liponsäure oder eines ihrer pharmazeutisch annehmbaren Derivate, Carnosin oder eines seiner pharmazeutisch annehmbaren Derivate und eine Zink-basierte Verbindung umfasst, wobei das Verhältnis zwischen α-Liponsäure oder eines ihrer pharmazeutisch annehmbaren Derivate und elementarem Zink von 5:1 bis 400:1 in Gewichtsteilen beträgt.

10. Zusammensetzung zur Verwendung gemäß wenigstens einem der vorhergehenden Ansprüche, die für jede tägliche Darreichungsform wenigstens einen pharmazeutisch annehmbaren Hilfsstoff umfasst.

11. Zusammensetzung zur Verwendung gemäß wenigstens einem der vorhergehenden Ansprüche, die für jede tägliche Darreichungsform, wenigstens ein Vitamin, wie beispielsweise Thiamin (Vitamin B1), Riboflavin (Vitamin B2), Pyridoxin (Vitamin B6), Pantothensäure (Vitamin B5), Cyanocobalamin (Vitamin B12), Niacin (Vitamin B3) und Folate umfasst.

12. Zusammensetzung zur Verwendung gemäß wenigstens einem der vorhergehenden Ansprüche in Form von Puder, Mikrogranulaten, Granulaten, Mikrokapseln, festen Kapseln, weichen Kapseln, Tabletten oder Pillen, Beuteln, wässrigen Lösungen, öligen Lösungen, Suspensionen oder Tropfen zum Einnehmen.

## Revendications

1. Composition comprenant de l'acide α-lipoïque ou un dérivé de celui-ci choisi parmi les sels de l'acide α-lipoïque avec des métaux alcalins, les sels de l'acide α-lipoïque avec des métaux alcalino-terreux, les sels de l'acide α-lipoïque avec des métaux de transition, un sel d'acide α-lipoïque avec le 2-amino-2-hydroxyméthylpropane-1,3-diol dans un rapport de 1:1, le lipoate de méthyle, le lipoate d'éthyle, ou des lipoamides et de la carnosine ou des dérivés pharmaceutiquement acceptables de celle-ci choisis parmi un sel de carnosine, l'ansérine, l'homocarnosine, la N-α-acétylcarnosine ou la carcinine, pour une utilisation dans le traitement du syndrome du membre fantôme.

2. Composition pour une utilisation selon la revendication 1, comprenant pour chaque quantité de dose journalière, de l'acide α-lipoïque ou un dérivé pharmaceutiquement acceptable de celui-ci dans une quantité de 150 à 1 400 mg et de la carnosine ou un dérivé pharmaceutiquement acceptable de celle-ci dans une quantité de 50 à 600 mg.

3. Composition pour une utilisation selon la revendication 2 comprenant, pour chaque quantité de dose journalière, de l'acide α-lipoïque ou un dérivé pharmaceutiquement acceptable de celui-ci dans une quantité de 350 à 900 mg et de la carnosine ou un dérivé pharmaceutiquement acceptable de celle-ci dans une quantité de 150 à 450 mg.

4. Composition pour une utilisation selon la revendication 1, 2 ou 3 comprenant, pour chaque quantité de
dose journalière, de l'acide α-lipoïque et de la carnosine ou des dérivés pharmaceutiquement acceptables de ceux-ci dans un rapport de 0,4:1 à 5:1 en parties en poids.

5. Composition pour une utilisation selon l'une quelconque des revendications 1 à 4, comprenant en outre une quantité efficace de composés à base de zinc.

6. Composition pour une utilisation selon la revendication 5 comprenant, pour chaque quantité de dose journalière, du zinc élémentaire dans une quantité de 1 à 100 mg.

7. Composition pour une utilisation selon la revendication 5 comprenant, pour chaque quantité de dose journalière, de l'acide α-lipoïque ou un dérivé pharmaceutiquement acceptable de celui-ci dans une quantité de 150 à 1 400 mg, de la carnosine ou un dérivé pharmaceutiquement acceptable de celle-ci dans une quantité de 50 à 600 mg, et un composé à base de zinc, la quantité de zinc élémentaire allant de 1 à 100 mg.

8. Composition pour une utilisation selon l'une quelconque des revendications 5 à 7 comprenant, pour chaque quantité de dose journalière, de l'acide α-lipoïque ou un dérivé pharmaceutiquement acceptable de celui-ci, de la carnosine ou un dérivé pharmaceutiquement acceptable de celle-ci et un composé à base de zinc, le rapport entre la carnosine ou un dérivé pharmaceutiquement acceptable de celle-ci et le zinc élémentaire allant de 5:1 à 50:1 en parties en poids.

9. Composition pour une utilisation selon l'une
quelconque des revendications 5 à 8 comprenant, pour chaque quantité de dose journalière, de l'acide α-lipoïque ou un dérivé pharmaceutiquement acceptable de celui-ci, de la carnosine ou un dérivé pharmaceutiquement acceptable de celle-ci et un composé à base de zinc, le rapport entre l'acide α-lipoïque ou un dérivé pharmaceutiquement acceptable de celui-ci et le zinc élémentaire allant de 5:1 à 400:1 en parties en poids.

10. Composition pour une utilisation selon l'une quelconque des revendications précédentes comprenant, pour chaque unité posologique journalière, au moins un excipient pharmaceutiquement acceptable.

11. Composition pour une utilisation selon l'une quelconque des revendications précédentes comprenant, pour chaque unité posologique journalière, au moins une vitamine, telle que, par exemple, la thiamine (vitamine B1), la riboflavine (vitamine B2), la pyridoxine (vitamine B6), l'acide pantothénique (vitamine B5), la cyanocobalamine (vitamine B12), la niacine (vitamine B3) et les folates.

12. Composition pour une utilisation selon l'une quelconque des revendications précédentes, sous forme de poudre, micro-granulé, granulé, microcapsule, gélule rigide, gélule molle, comprimé ou pilule, sachet, solution aqueuse, solution huileuse, suspension ou gouttes orales.
